Europäisches Patentamt

European Patent Office

Office européen des brevets

(19)

(11) Publication number: **0 252 927 B1**

(12)

# EUROPEAN PATENT SPECIFICATION

(45) Date of publication of patent specification :
24.07.91 Bulletin 91/30

(51) Int. Cl.$^5$ : **G01N 15/00, G01N 11/02**

(21) Application number : **86906700.9**

(22) Date of filing : **30.10.86**

(86) International application number :
**PCT/US86/02334**

(87) International publication number :
**WO 87/04245 16.07.87 Gazette 87/15**

(54) DETERMINING FLOW PROPERTIES OF PARTICULATE MATERIALS.

(30) Priority : **31.12.85 US 815226**

(43) Date of publication of application :
**20.01.88 Bulletin 88/03**

(45) Publication of the grant of the patent :
**24.07.91 Bulletin 91/30**

(84) Designated Contracting States :
**AT BE CH DE FR GB IT LI LU NL SE**

(56) References cited :
**US-A- 2 633 027
US-A- 3 221 560
US-A- 3 890 830
US-A- 4 274 286
Powder Technology, vol. 4, no. 6, 22 February
1971 (Switzerland), J.C. Williams et al., "The
direct measurement of the failure function of a
cohesive powder", pages 328-337**

(73) Proprietor : **JR JOHANSON, INC.**
**2975 Hawk Hill Lane**
**San Luis Obispo, CA 93401 (US)**

(72) Inventor : **JOHANSON, Jerry, Ray**
**2975 Hawk Hill Lane**
**San Luis Obispo, CA 93401 (US)**
Inventor : **JOHANSON, Kerry, Dee**
**2975 Hawk Hill Lane**
**San Luis Obispo, CA 93401 (US)**

(74) Representative : **Wagner, Karl H.**
**WAGNER & GEYER Patentanwälte**
**Gewürzmühlstrasse 5 Postfach 246**
**W-8000 München 22 (DE)**

## Description

The present invention is in the field of bulk particulate solids and more specifically relates to an apparatus and testing method for determining on the basis of bench scale testing whether particulate matter will flow under the action of gravity through an outlet in the bottom of a container.

Background Art

Bulk solids in a divided state such as flour, sugar, ores, dry chemicals and coal are generally handled in silos or containers that require gravity flow for discharge. One of the problems of designing such containers is sizing of the outlet so that the solids do not form an obstruction by arching across the outlet.

The size of outlet required to prevent arching is a function of the unconfined yield strength of the material, the density of the material, and the shape of the outlet.

There are basically two methods for determining this critical dimension. First, one could construct a full size hopper, load it with the material, and observe whether flow takes place from the outlet. The other approach known in the art is to measure the unconfined yield strength, and then to use this unconfined yield strength as a function of consolidation pressure to analyze the results to predict the hopper outlet dimension. This latter approach has been described in the following technical papers : Jenike, A. W., P. J. Elsey, and R. H. Woolley. "Flow Properties of Bulk Solids." Proc. Amer. Soc. Test. Mater. 60 : 1168-1181, 1960 ; Jenike, A. W. "Gravity Flow of Bulk Solids." Univ. of Utah Engineering Experiment Station Bulletin No. 108, 1961 ; Johanson, J. R. "Know Your Material—How to Predict and Use the Properties of Bulk Solids." Chem. Eng., october 30, 1978, pp. 9-17.

This latter method is usually accomplished by means of a direct shear tester and therefore it is rather cumbersome and time-consuming to obtain the results. A quicker and easier method is needed, particularly for use in the field, where portable testing equipment would be especially handy.

US-A-2 633 027 discloses a method of testing flow characteristics of granular materials by confining a predetermined amount of a granular material within a restricted space on a support, applying a predetermined pressure on the material in a direction to pack it in the restricted space, removing the pressure from the material, removing the support on which the material is positioned to determine whether any portion of the material is bridged across the restricted space, and repeating the above-outlined steps with incremental increases in the pressure applied to the material until a portion of the material is packed sufficiently to cause it to bridge across the space, whereby the packing point of the material is determined.

In Powder Technology, vol. 4, no. 6, 22 February 1971 ; Williams, J. C. et al., "The direct Measurement of the Failure Function of a Cohesive Powder", pages 328-337, the flow function of a powder has been determined directly by compacting a cylindrical specimen and determining the unconfined yield strength. An experimental method has been developed for eliminating the effect of wall friction during compaction.

Disclosure of the Invention

It is an object of the present invention to provide apparatus and a bench scale test method to determine whether flow will occur through a given outlet under the action of gravity alone.

It is a further object to provide apparatus and a test method for determining the unconfined yield strength of a particulate material.

A further objective is to provide an apparatus and test method that is applicable to conical containers as well as to containers of rectangular horizontal cross section.

These objectives are accomplished by a novel test chamber. The portion of the test chamber in which the material is tested includes no cylindrical walls that contact the material, and in this way the uncertainty associated with the frictional effects of cylindrical walls is eliminated. The side walls of the test chamber conform to the surface of a cone, and the larger end of the conical section is closed by a plug that includes an inwardly facing coaxial concave surface. This unique shape of the test chamber affords minimum interference with the plastic stress field present in the material. During the consolidation phase of the test, the conical walls provide an increasing cross sectional area in the direction of motion so as to minimize the frictional effects of the walls. During the failure phase of the test, the same conical walls provide a decreasing cross sectional area in the direction of motion to insure an arching condition similar to that occurring in a hopper during failure conditions.

In a novel aspect of the testing method, the material to be tested is consolidated in the test cell with the concave plug at the bottom of the test cell, and then the entire test cell including the concave plug is turned upside down for the failure loading portion of the test.

These and other objectives and advantages of the present invention will become clear from the detailed description given below in which several preferred embodiments are described in relation to the drawings. The detailed description is presented to illustrate the present invention, but is not intended to limit it.

## Brief Description of the Drawings

Figure 1 is an elevational view in cross section showing the apparatus of a preferred embodiment of the present invention at the initial consolidation phase of the test procedure ;

Figure 2 is an elevational view in cross section showing the apparatus at a later stage of the test procedure;

Figure 3 is an elevational view in cross section showing the apparatus at a later stage of the test procedure;

Figure 4 is a perspective view showing a rectangular test apparatus ;

Figure 5 is an elevational view in cross section showing the apparatus of a second preferred embodiment of the present invention at the initial consolidation phase of the test procedure ; and,

Figure 6 is an elevational view in cross section showing the apparatus of Figure 5 at the start of the failure loading phase of the test procedure.

## Best Mode for Carrying Out the Invention

In the first part of this section, the structure of the apparatus will be described in detail. Next, the methods of using the apparatus in carrying out various tests will be discussed. Finally, several alternative embodiments and variations will be described.

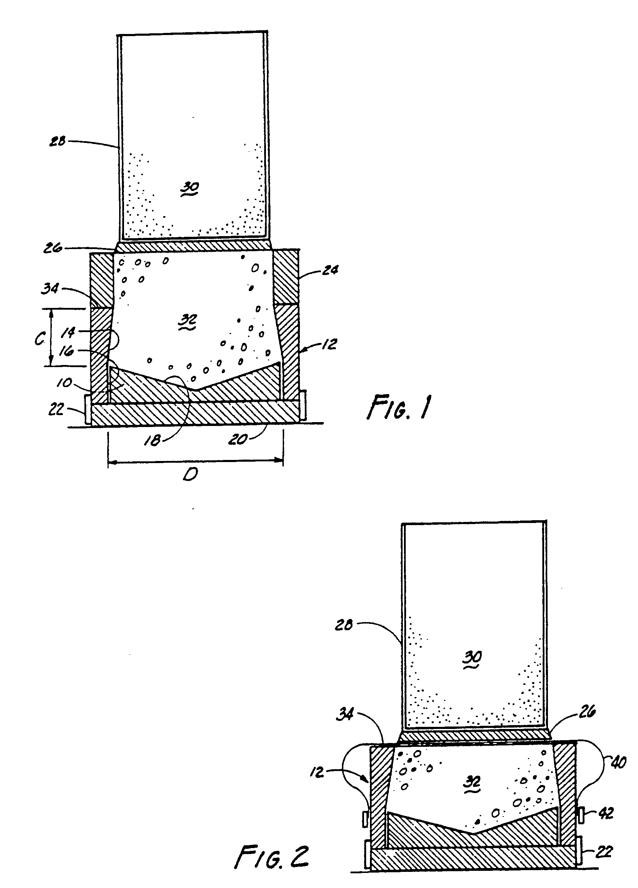

Turning now to the drawings in which like parts are denoted by the same reference numeral throughout, there is shown in Figure 1 a preferred embodiment of the apparatus that is used for performing the tests described below.

The apparatus includes a consolidation base 20 which rests on the floor or on a bench. The conical plug 10 rests on the base 20 with its conical surface 18 facing the inside of the test cell 12. The shape of the space enclosed by the test cell 12 and by the conical plug 10 is an important aspect of the present invention, as will be described below.

The test cell 12 is a unitary sleeve-like part having a conical inside surface 14 shaped to conform to a truncated cone joined at its larger diameter to a cylindrical inside surface 16. The conical inside surface 14 is inclined to the vertical by approximately six degrees, so that it could also be said that the cone to which the surface 14 conforms has a vertex angle of twelve degrees, in the preferred embodiment. If the semi-angle is less than four degrees, the convergence will be insufficient to form an arch during the failure step of the test procedure. On the other hand, the angle must not be so great that it becomes incompatible with the mass flow stress field in the test cell during the failure step of the test procedure. The conical plug 10 is slightly smaller in diameter than the cylindrical inside surface 16 so that the plug 10 can, at a later stage of the test, move freely in the axial direction with respect to the test cell 12. The height of the conical plug 10 in the axial direction is slightly less than the height of the cylindrical inside surface 16 to accommodate some movement by the plug in the axial direction.

The apparatus further includes a mold ring 24 that rests upon the test cell 12 and that has a diameter approximately equal to the diameter of the opening at the top 34 of the test cell.

In a typical test, the test cell 12 and the mold ring 24 are filled with the material 32 under test, and the material 32 is then consolidated to a specific degree.

In the best mode for carrying out the invention, the consolidation is accomplished by placing the flat disk 26 on top of the material 32 under test. The diameter of the flat disk is slightly less than the inside diameter of the mold ring 24 to permit the disk 26 to move downwardly into the mold ring 24 without contacting the mold ring. In the best mode, a consolidation load container 28 is then rested upon the disk 26 and is slowly filled with a particulate ballast material 30 to a specific pre-calculated depth H. All of the parts of the apparatus are made of steel in the preferred embodiment, although in other embodiments any strong durable non-absorbent material may be used.

Because the material in the full-size hopper typically includes a degree of moisture, the material 32 under test also contains moisture, and it is important to prevent the loss of this moisture, since it affects the accuracy of the results. For this reason, a metal or rubber keeper ring 22 tightly encircles the crack between the test cell 12 and the base 20.

The length of time during which the consolidation load is applied should approximate the dead time of the material in the proposed hopper, and if this amounts to an appreciable amount of time, it may be essential to take the additional step against moisture loss shown in Figure 2. In Figure 2, a moisture seal 40 is inserted between the material 32 under test and the disk 26.

The moisture seal 40 consists of a thin sheet of pliable plastic, which is held in place by the keeper 42.

EP 0 252 927 B1

The keeper 42 in a preferred embodiment consists of a band of metal or rubber.

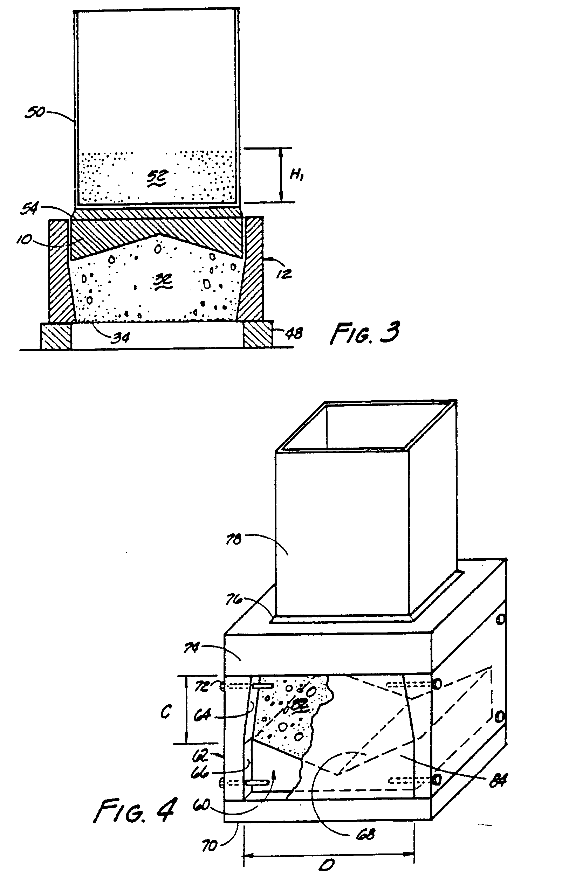

Figure 3 shows the apparatus at a later stage of the test method. After the material being tested has consolidated for the desired length of time, the moisture seals are removed and the test cell 12 is turned upside down and placed on the failure base 48. The failure base 48 is merely a support which does not interfere with the material in the test cell or prevent the material 32 from falling out of the test cell. Note that in Figure 3, the test cell 12 is upside down relative to its position in Figures 1 and 2, and that the conical plug 10 remains in place in the test cell. On top of the conical plug is placed the failure disk 54 which, in a preferred embodiment, is a disk of steel which serves to evenly distribute the applied failure load. The failure load container 50, which is not necessarily the same as the consolidation load container 28, is placed on top of the failure disk 54 and is gradually filled with a ballast material, such as sand, gravel, or water. At some point as additional failure load material 52 is added, the pressure loading on the material 32 becomes too great to resist, and the material 32 collapses and falls out of the test cell 12. The height $H_1$ of the failure load material 52 at the instant of failure is carefully noted and, as will be seen below, is used in calculating the test results.

It will be recognized that the conical shape of the test cell is an important aspect of the invention. During the consolidation phase of testing, shown in Figures 1 and 2, this shape provides an increase in area in the direction of flow, insuring a minimum of frictional reaction from the walls of the test cell during the consolidation phase, thereby permitting a nearly uniform and known compaction of the material tested. During the failure phase of testing, shown in Figure 3, the conical shape of the test cell provides a decreasing cross sectional area in the direction of flow so as to simulate the condition occurring in a full-scale hopper.

The apparatus shown in Figures 1-3 is used for determining whether a particulate material in a large container will flow by the action of gravity alone through a circular outlet in the bottom of the container. If, instead of being circular, the outlet is rectangular, the apparatus shown in Figure 4 may be used. That apparatus is in every sense the rectangular analog of the apparatus shown in Figures 1-3.

In the apparatus of Figure 4, a trough-shaped plug 60 having the inclined plane faces 68 extends into the test cell 62. The test cell 62 is rectangular in horizontal cross-section. The right and left walls as shown in Figure 4 each include a vertical portion 66 and an inclined portion 64. The front and rear walls are vertical only, and are provided with removable panels 84 that are held in place by removable pins of which the pin 72 is typical.

Figure 4 is comparable to Figure 1 in that it shows the apparatus in the initial stage of the testing. A rectangular mold frame 74 rests on top of the test cell 62, and is comparable to the mold ring 24 of Figure 1. A consolidation plate 76 is interposed between the consolidation load container 78 and the material 82 under test. The entire apparatus rests on a consolidation base 70.

With reference to the apparatus of Figures 1-4 inclusive, it is essential when testing cohesive solids (defined as those in which the ratio R of the compacting pressure to the instantaneous unconfined yield stress is less than 2.0) that the walls of the converging parts of the test cell have a rough finish and that the height-to-diameter ratio C/D should not exceed 0.2 R. If the ratio exceeds 0.2 R, there is a considerable likelihood that recompaction of the material will occur during failure, thereby giving a false reading. Since the ratio R is seldom less than 1.1 for computing pressures of practical interest, in the preferred embodiment, C/D has been chosen to equal 0.22. For the embodiment shown in Figure 4, the critical value of C/D is about twice that for the configurations of Figures 1-3, and in a preferred embodiment, the ratio C/D is approximately equal to 0.4 R or 0.44 for R = 1.1.

This relatively larger allowable value of C for the rectangular test cell can be very important when testing solids with large particles where C must be several times larger than the particle size.

The test procedure is substantially the same for the embodiments of Figures 1 and 4. The apparatus shown and described, when used in accordance with the test procedure given below, can be used to predict whether a circular or rectangular outlet in the bottom of a container partially filled with a particulate material will discharge under the action of gravity alone or whether, instead, arching of the material above the outlet will develop to prevent the desired flow. It will be seen below, that in addition to determining whether or not flow will occur, the method can be used to determine the size of outlet required to prevent arching and, with some minor modifications, can be used to measure the unconfined yield strength of the material under test.

Initially, the test cell 12 with the conical plug 10 are resting on the base 20. The mold ring 2d is assumed to be resting on the test cell 12. The test cell is filled with the material under test to the top of the mold ring. Next, the disk 26 is placed on top of the material 32, the consolidation load container 28 is placed on top of the disk 26, and the consolidation load 30 is poured into the container 28. This causes the material 32 to consolidate, and if the material 32 has consolidated to a level below the top 34 of the test cell, it is necessary to remove the consolidation container, to refill the mold ring 24, and to apply the consolidation load 30 again to the material 32 until the consolidation level does not sink below the top 34 of the test cell.

Next, the mold ring 24 is removed and the material 32 is scraped level with the top 34 of the test cell. Thereafter, the disk 26 and the consolidation load container 28 are replaced as shown in Figure 2, and the consoli-

4

dation load 30 is applied and allowed to sit for a time equal to the time at rest of the material within the container being simulated. If this time at rest is in excess of a few minutes, it is necessary to cover the test cell with a moisture impermeable seal such as the moisture seal 40 shown in Figure 2.

When the apparatus is used to test whether or not flow will occur, the appropriate height H of ballast in the consolidation load container 28 is :

$$H = \frac{F}{1 + m} \frac{\gamma_1}{\gamma_2} \frac{A_1}{A_2} B - \frac{W_c}{\gamma_2 A_2}$$

where

$\gamma_1 =$     bulk density of material in test cell
$\gamma_2 =$     bulk density of ballast material
$A_1 =$     average cross sectional area of test cell
$A_2 =$     cross sectional area of consolidation load container
$W_c =$     weight of the consolidation load container
$B =$     diameter of circular outlet of a conical container or width of rectangular outlet of a rectangular container
$m =$     1 for a conical container
$m =$     0 for a rectangular container
$F =$     overpressure factor

The overpressure factor F accounts for the additional pressure caused by forces other than gravity that may be operative on the container being simulated.

Examples include vibration, the impact of a falling stream of particles entering the container, and the presence of gas pressure gradients within the material in the container such as might be caused by a flow of gas through the solids from top to bottom.

If vibrations cause a peak vertical acceleration of a, then

$$F = (1 + \frac{a}{g})$$

where g is the acceleration of gravity.

A downwardly acting gas pressure gradient of magnitude dp/dx can be accounted for by setting

$$F = \left(1 + \frac{dp/dx}{\gamma}\right)$$

where $\gamma$ is the bulk density of the material in the container and dp/dx is the pressure gradient in the upward direction.

The appropriate expression for F in the presence of a falling stream of particles is derived in "New Design Criteria for Hoppers and Bins" by J. R. Johanson and H. Colijn Iron & Steel Engineer, October 1964, pp. 85-104.

The consolidation pressure is given by

$$\sigma = F \frac{\gamma_1 B}{1 + m}$$

and the consolidation load is

$$L_c = F \frac{\gamma_1 A_1 B}{1 + m} = W_c + \gamma_2 A_2 H$$

After the consolidation time has elapsed, the consolidation load container 28, the disk 26, and the moisture seal 40 are removed and the test cell 12 along with the base 20, and including the conical plug 10 are turned upside down and placed on the failure base 48 in the position shown in Figure 3. Assuming the material 32 has a measurable cohesion, it should not fall out of the test cell in response to the shocks normally encountered in handling the apparatus, although clearly, shocks are to be avoided. Thereafter, the base 20 is removed and the failure disk 54 and the failure load container 50 are stacked on top of the conical plug 10 as shown in Figure 3. It is important that the disk 54 and the container 50 do not touch the test cell during the remaining phases of the test.

Next, the failure load 52 is very gradually poured into the failure load container 50 until failure of the material in the load cell occurs. Typically, such failures are rather abrupt and most of the material within the test cell will fall out upon failure. Very little actual movement of the conical plug 10 occurs before failure, and this limited movement is permitted by the clearance between the conical plug 10 and the inside of the test cell. The height $H_1$ of the material 52 within the failure load container 50 at the time of failure is determined by leveling the material in the failure load container 50 and measuring the height $H_1$ shown in Figure 3.

If this value of height $H_1$ satisfies the following inequality, then flow will occur by gravity from the outlet of diameter B ;

$$H_1 \leq (\frac{B}{D} - 1 - \frac{W_c}{CA_1\gamma_1})C \frac{A_1}{A_2} \frac{\gamma_1}{\gamma_2}$$

where C is the height of the test cell and D is the diameter of the test cell, as shown in Figure 1.

As an alternative to increasing the value of $H_1$, the force $L_F$ applied to cause failure could be measured and the critical failure condition could be described by the equation :

$$L_f \leq (\frac{B}{D} - 1) CA_1\gamma_1$$

The test procedure for the rectangular opening using the apparatus of Figure 4 is identical to the procedure just described with the exception that the value of m is different from that used for the circular opening, and the symbol B is defined to be the width of the rectangular outlet which has vertical inlet walls or is infinitely long, and D is the width of the rectangular test cell. It is seen that these differences affect only the magnitude of the consolidation load and the calculated value of $H_1$, but do not otherwise affect the steps of the test procedure.

Sometimes it is desirable to measure the unconfined yield strength of the material, and this can be done using a relatively minor variation on the above test procedure. The only differences where the unconfined yield strength is to be determined are that the applied consolidation pressure should equal

$$\sigma_c = \frac{L_c}{A_1} + \gamma_1 \frac{C}{2}$$

where the symbols have the same meaning as above, and if the applied load at failure is denoted by $L_F$ then the unconfined yield strength $f_c$ may be calculated by use of the equation

$$f_c = \frac{D}{h} (\gamma_1 + \frac{L_f}{A_1C})$$

where h is 2.1 for the conical test cell and h is 1.1 for the rectangular test cell.

In another minor variation on the method for determining whether bulk solids will or will not flow from an outlet in a container, it is recognized that there are other techniques for applying a consolidation pressure to the material 32 in the test cell. For example, a pneumatic or hydraulic ram could be used, or even a screw jack could be used with a load cell to indicate the force applied. Regardless of the device used to apply the consolidation pressure, the consolidation pressure should equal

$$\sigma_c = F \frac{\gamma_1 B}{1 + m}$$

Likewise the pressure at the time of failure can be determined in other ways than using the failure load container 50 and the failure load 52 of Figure 3. Alternative devices for applying the failure pressure can be employed consistent with the test method, and so long as the measured pressure at failure does not exceed the following quantity, then flow will occur by gravity alone :

$$\sigma_F = (\frac{B}{D} - 1) \, C\gamma_1$$

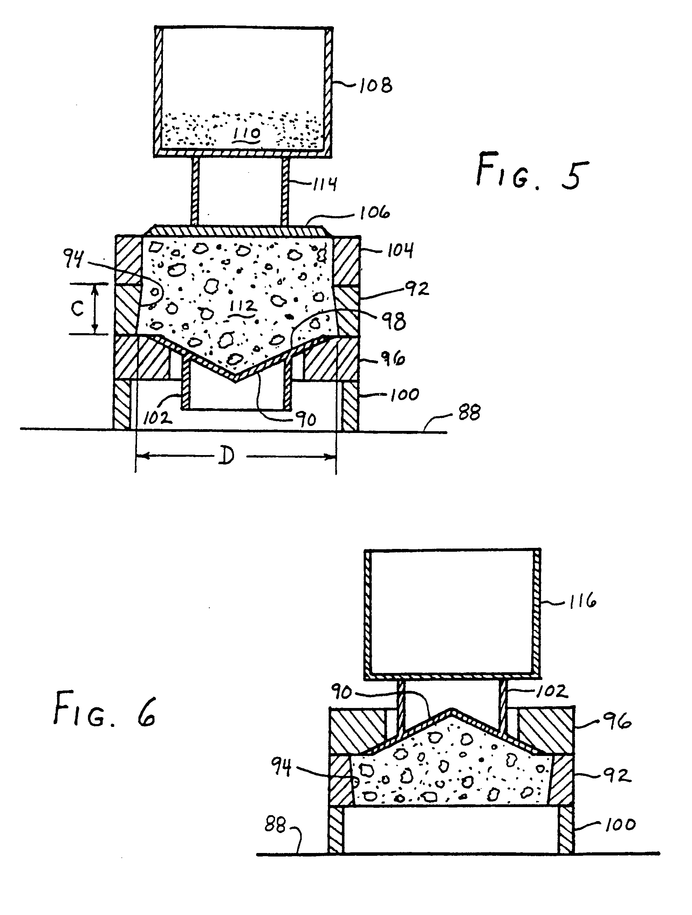

A second preferred embodiment is shown in Figure 5 and 6, during the consolidation phase and at the beginning of the failure phase of the test procedure respectively. In this embodiment, the other parts of the apparatus are supported above the floor 88 or bench top by a bottom spacer ring 100.

The test cell in this embodiment includes a ring 92 that has a conical inner surface 94. During the consolidation phase, the ring 92 is supported on the plug support ring 96, which in turn rests on the bottom spacer ring 100. As shown in the drawings, the height of the ring 92 is C, and D represents the diameter of the larger end of the conical surface 94.

The plug 90, which in one version of this embodiment includes the cylindrical spacer 102, spans the larger diameter end of the conical surface 94 and rests on a beveled portion of the plug support ring 96. In addition to supporting the plug 90, the beveled portion of the ring 96 centers the plug 90. The concave conical surface 98 of the plug 90 faces the interior of the test chamber. In accordance with the present invention, the inwardly-facing surface of the plug 90 must be concave, but it does not necessarily have to be conical.

A mold ring 104 rests on the ring 92. The inside surface of the mold ring is cylindrical and its diameter is the same as the diameter of the smaller end of the conical surface 94, so that the mold ring 104 forms an upward extension of the ring 92.

The consolidation pressure is applied, in one version of this embodiment, by applying a weight to the consolidation disk 106 that rests on the particulate material 112. The disk 106 has a diameter slightly smaller than the inside diameter of the mold ring 104, to permit the disk 106 to move freely in the axial direction as the material 112 compacts. In this version of the embodiment, a spacer 114 supports a consolidation load container 108 a short distance above the disk 106. The consolidation load may be increased by adding consolidation ballast 110 to the consolidation load container 108.

During the consolidation phase, the direction of movement of the particles is downward toward the plug 90. The downwardly-diverging shape of the conical surface 94 prevents friction between that surface and the particulate material from interfering with the movement of the particles.

The consolidation load should be applied to the particulate material in the test cell for an interval of time approximately equal to the time at rest of the particulate material in the full-size hopper. If the material is moist and if the time is long, a moisture retaining membrane similar to the membrane 40 of Figure 2 may be employed to prevent loss of moisture.

At the end of the consolidation phase, the load is removed. Next the mold ring 104 is removed and the consolidated material above the ring 92 is removed.

Thereafter, the rings 92 and 96 along with the plug 90 are inverted, care being taken to prevent relative movement of the rings 92 and 96, and to avoid bumping the exposed portions of the plug 90. The ring 92 is gently lowered onto the bottom spacer ring 100, so that the apparatus then has the configuration shown in Figure 6. The failure load container 116, which may be identical to the consolidation load container 108, is then placed on the spacer 102. The load on the particulate material is gradually increased by adding ballast to the failure load container 116, until failure occurs. The load at which failure occurs is then noted.

Only a small downward movement of the plug 90 occurs before failure, and the failure is catastrophic, with the particulate material abruptly falling from the ring 92 onto the floor 88 or bench top. To accommodate this small motion of the plug 90, the outside diameter of the plug 90 is slightly less than the diameter of the larger-diameter end of the surface 94. This is shown somewhat exaggerated in Figures 5 and 6 for clarity. Likewise, the wall thickness of the plug 90 is exaggerated in the drawings.

During the failure phase of the test procedure, the downwardly converging surface 94 simulates the downwardly converging walls of the full-size hopper.

As was the case with the first preferred embodiment described above, by the use of appropriate pre-calculated consolidation loads, any of several flow-related properties of the particulate material may be ascertained by use of the apparatus of this second preferred embodiment. The equations for the apparatus of this second preferred embodiment are identical to those used in the first preferred embodiment described above.

Thus, there has been described a novel apparatus for scale-model testing to determine whether or not flow will occur under the action or gravity from a circular or a rectangular outlet in the bottom of a container that is partly filled with particulate matter. The shape of the space within the test cell is of particular significance. The test chamber walls conform to the surface of a cone, and the end of the test chamber is plugged by a movable plug having a concave inwardly-facing surface. Accordingly, the material under test never contacts a cylindrical wall and this reduces test error caused by the unknown frictional forces that come into play when the material contacts a cylindrical wall. Also, the shape of the test cell is designed to not interfere with the plastic stress field that develops in the sample during testing. Accordingly, the novel shape of the test cell is highly advantageous and results in greater accuracy.

There has also been described in detail a scale model test procedure that allows accurate prediction of whether or not an outlet in the bottom of a container of particulate matter will discharge under the action of gravity alone. This method, which requires the use of the special test apparatus involves the steps of consolidating the material to a specific extent by application of a specific pressure to it and then inverting the test cell prior to determining the pressure required to cause failure of the material.

From the above it is clear that the apparatus for use in ascertaining the flow properties of a particulate material can be said to comprise a hollow test cell that includes a frustrated wedge section defined by the inwardly-facing surface 14 conforming to a section of a wedge, said frustrated wedge section having a smaller end and a larger end ; and, the plug 60 which is located adjacent to and spanning the larger end of the frustrated wedge section, but is slightly narrower than it, so as to be freely movable a limited distance into the space within the frustrated wedge section. Preferably the vertex semi-angle of the frustrated wedge section is in the range between a minimum of 4 degrees and a maximum of $\theta_p$, where $\theta_p$ is the largest angle compatible with the mass-flow stress field in the test cell ; or, expressed differently, the vertex semi-angle of the conical section (having a conical inside surface 14) is in the range between a minimum of 4 degrees and a maximum of $\theta_c$, where $\theta_c$ is the largest angle compatible with the mass-flow stress field in the test cell.

## Industrial Applicability

The method and apparatus described above is most helpful in predicting the behavior of full-scale containers of particulate materials from small-scale laboratory bench tests. In this way the success of a hopper design can be assured and expensive mistakes can be avoided. The method is applicable to all kinds of particulate materials, from the finest powders to coarse ores, so that the potential applications are innumerable.

## Claims

1. A method of bench scale testing to ascertain a flow-related property of a particulate material, comprising the steps of :

a) providing a hollow test cell that includes a conical section defined by an inwardly-facing surface conforming to a section of height C of a cone, the conical section having a smaller-diameter end and a larger-diameter end of diameter D, and that further includes a plug located adjacent to and spanning the larger-diameter end of the conical section, but slightly smaller in diameter than it, so as to be freely movable axially a limited distance into the space within the conical section, the plug having a concave inwardly-facing surface ;

b) placing the test cell on a supporting surface with the larger-diameter end of the conical section below its smaller-diameter end and with the plug supported in its aforementioned location adjacent the larger-diameter end of the conical section ;

c) filling the test cell completely full with the particulate material ;

d) consolidating the particulate material within the test cell by applying a downward pressure at the smaller-diameter end of the conical section, so that the direction of motion of the particulate material during consolidation is toward the plug ;

e) inverting the test cell with the plug and the consolidated particulate material still in it, so that after the inversion the larger-diameter end of the conical section is above its smaller-diameter end ;

f) applying a gradually-increasing downward load to the plug to produce failure motion of the particulate material toward the smaller-diameter end of the conical section ; and,

g) noting the downward load at which failure occurs.

2. The method of Claim 1 wherein the flow-related property is the minimum diameter $B_{MIN}$ of an outlet in the bottom of a full-size container at which flow of the particulate material will occur by gravity alone ; and wherein the step of consolidating the particulate material is further characterized by the step of applying a pressure to the particulate material at the smaller-diameter end of the conical section equal to the consolidation pressure at the corresponding location in the full-size container ; and wherein, following the step of noting the downward load, the method is further characterized by the step of calculating the minimum diameter at which flow will occur by the equation

$$B_{MIN} = \frac{L_F D}{C A_1 \gamma_1} + D$$

where

$L_F$     is the noted downward load,

$A_1$     is the average cross sectional area of the test cell, and

$\gamma_1$     is the bulk density of the particulate material.

3. The method of Claim 2 wherein the particulate material has been at rest in the full-size container for an appreciable time, and wherein the step of consolidating is further characterized by applying the consolidation pressure for an interval of time equal to how long the particulate material in the full-size container has been at rest.

4. The method of Claim 1 wherein the flow-related property is whether or not the particulate material will flow, after having been at rest for some time, from a full-size container that requires gravity flow for discharge and that has an outlet of diameter B in its bottom ; and wherein the step of consolidating the particulate material within the test cell by applying a downward pressure is further characterized by the step of applying a consolidation load $L_c$ uniformly distributed over the particulate material at the smaller-diameter end of the conical section, so that the direction of motion of the particulate material during consolidation is toward the plug, where

$$L_c = \frac{1}{2}\gamma_1 A_1 B$$

where

$F =$     overpressure factor,

$\gamma_1 =$     bulk density of the particulate material being tested,

$A_1 =$     average cross sectional area of the test cell ;

and wherein the method of Claim 1 is further characterized by the step, following the step of noting the downward load, of determining whether the noted load at which failure occurred is less than

$$L_F = (\frac{B}{D} - 1)\, CA_1\gamma_1$$

whereby, if it is, then flow from the full-size container will occur by gravity, but if it is not, then flow from the full size container will not occur by gravity.

5. The method of Claim 4 wherein the consolidation load $L_c$ is applied to the particulate material for an interval of time equal to how long the particulate material in the full-size container has been at rest.

6. The method of Claim 1 wherein the flow-related property is whether or not the particulate material will flow, after having been at rest for some time, from a full-size container that requires gravity flow for discharge and that has an outlet of diameter B in its bottom ; and before the step of filling the test cell, further characterized by the step of providing a mold ring that is hollow and that has a cylindrical inside surface of diameter equal to the diameter of the smaller-diameter end of the conical section ; and before the step of filling the test cell, further characterized by the step of resting the mold ring on top of and coaxial with the test cell to form an upward extension of it ; and after the step of filling the test cell, further characterized by the steps of filling the mold ring with the particulate material and placing a consolidation load disk having an outside diameter slightly less than the inside diameter of the mold ring on top of the particulate material in the mold ring ; and wherein the step of consolidating the particulate material is accomplished by applying a consolidation load $L_c$ uniformly distributed over the top of the particulate arterial in the mold ring by the consolidation load disk, so that the direction of motion of the particulate arterial during consolidation is toward the plug, where

$$L_c = \frac{1}{2} F\gamma_1 A_1 B$$

and

$F =$     overpressure factor,

$\gamma_1 =$     bulk density of the particulate material being tested,

$A_1 =$     average cross sectional area of the test cell ;

and wherein, before the step of inverting the test cell, the method is further characterized by the steps of continuing to apply the consolidation load for an interval of time equal to how long the particulate material in the full-size container has been at rest, and removing the mold ring and the particulate material contained in it from the test cell ; and after the step of noting the downward load, the method is further characterized by the step of determining whether the noted load at which failure occurred is less than

$$L_f = (\frac{B}{D} - 1)\, CA_1\gamma_1$$

whereby, if it is, then flow from the full-size container will occur by gravity, but if it is not, then flow from the full size container will not occur by gravity.

7. The method of Claim 1 wherein the flow-related property is the unconfined yield strength of the particulate material and wherein, in the consolidating step the downward pressure $\sigma_c$ is given by

$$\sigma_c = \frac{L_c}{A_1} + \frac{1}{2}\gamma_1 C$$

where

$L_c =$     consolidation load
$A_1 =$     average cross sectional area of the test cell
$\gamma_1 =$     bulk density of the particulate material ;

and, after the step of noting the load, the method is further characterized by the step of calculating the unconfined yield strength by the equation

$$f_c = \frac{D}{h} (\gamma_1 + \frac{L_F}{A_1 C})$$

where h = 2.1.

8. Apparatus for use in ascertaining the flow properties of a particulate material, comprising : a hollow test cell that includes a conical section defined by an inwardly-facing surface conforming to a section of a cone, said conical section having a smaller-diameter end and a larger-diameter end ; and, a plug located adjacent to and spanning the larger-diameter end of said conical section ; but slightly smaller in diameter than it, so as to be freely movable axially a limited distance into the space within said conical section, said plug having a concave inwardly-facing surface.

9. The apparatus of Claim 8 wherein the vertex semi-angle of the conical section is in the range between a minimum of 4 degrees and a maximum of $\theta_c$, where $\theta_c$ is the largest angle compatible with the mass-flow stress field in the test cell.

10. A method of bench scale testing to ascertain a flow-related property of a particulate material, comprising the steps of :

a) providing a hollow test cell that includes a frustrated wedge section defined by an inwardly-facing surface conforming to a section of height C of a wedge, the frustrated wedge section having a smaller end and a larger end of width D, and that further includes a plug located adjacent to and spanning the larger end of the frustrated wedge section ; but slightly narrower than it, so as to be freely movable a limited distance into the space within the frustrated wedge section ; the plug having a concave trough-like inwardly-facing surface ;

b) placing the test cell on a supporting surface with the larger end of the frustrated wedge section below its smaller end and with the plug supported in its aforementioned location adjacent the larger end of the frustrated wedge section ;

c) filling the test cell completely full with particulate material ;

d) consolidating the particulate material within the test cell by applying a downward pressure at the smaller end of the frustrated wedge section, so that the direction of motion of the particulate material during consolidation is toward the plug ;

e) inverting the test cell with the plug and the consolidated particulate material still in it, so that after the inversion the larger end of the frustrated wedge section is above its smaller end ;

f) applying a gradually-increasing downward load to the plug to produce failure motion of the particulate material toward the smaller end of the frustrated wedge section ; and,

g) noting the downward load at which failure occurs.

11. The method of Claim 10 wherein the flow-related property is the minimum width $B_{MIN}$ of an outlet in the bottom of a full-size container at which flow of the particulate material will occur by gravity alone ; and wherein the step of consolidating the particulate material is further characterized by the step of applying a pressure to the particulate material at the smaller-diameter end of the conical section equal to the consolidation pressure at the corresponding location in the full-size container ; and wherein, following the step of noting the downward load, the method is further characterized by the step of calculating the minimum width at which flow will occur by the equation

$$B_{MIN} = \frac{L_F D}{CA_1 \gamma_1} + D$$

where

$L_F$     is the noted downward load,

$A_1$     is the average cross sectional area of the test cell, and

$\gamma_1$     is the bulk density of the particulate material.

12. The method of Claim 11 wherein the particulate material has been at rest in the full-size container for an appreciable time, and wherein the step of consolidating is further characterized by applying the consolidation pressure for an interval of time equal to how long the particulate material in the full-size container has been at rest.

13. The method of Claim 10 wherein the flow-related property is whether or not the particulate material will flow, after having been at rest for some time, from a full-size container that requires gravity flow for discharge and that has an outlet of width B in its bottom ; and wherein the step of consolidating the particulate material within the test cell by applying a downward pressure is further characterized by the step of applying a consolidation load $L_c$ uniformaly distributed over the particulate arterial at the smaller end of the frustrated wedge section, so that the direction of motion of the particulate material during consolidation is toward the plug, where

$$L_c = F \gamma_1 A_1 B$$

where

F =       overpressure factor,

$\gamma_1$ =       bulk density of the particulate material being tested,

$A_1$ =       average cross sectional area of the test cell ;

and wherein the method of Claim 11 is further characterized by the step, following the step of noting the downward load, of determining whether the noted load at which failure occurred is less than

$$L_F = (\frac{B}{D} - 1) CA_1 \gamma_1$$

whereby, if it is, then flow from the full-size container will occur by gravity, but if it is not, then flow from the full-size container will not occur by gravity.

14. The method of Claim 13 wherein the consolidation load $L_c$ is applied to the particulate material for an interval of time equal to how long the particulate material in the full-size container has been at rest.

15. The method of Claim 10 wherein the flow-related property is whether or not the particulate material will flow, after having been at rest for some time, from a full-size container that requires gravity flow for discharge and that has an outlet of width B in its bottom ; and before the step of filling the test cell, further characterized by the step of providing a mold frame that is hollow and that has a rectangular inside surface of dimensions equal to those of the smaller end of the frustrated wedge section ; and, before the step of filling the test cell, further characterized by the step of resting the mold frame on top of and registered with the test cell to form an upward extension of it ; and, after the step of filling the test cell, further charaterized by the steps of filling the mold ring with the particulate material and placing a consolidation load plate having outside dimensions slightly

11

less than the inside dimensions of the mold frame on top of the particulate material in the mold frame ; and wherein the step of consolidating the particulate material is accomplished by applying a consolidation load $L_c$ uniformly distributed over the top of the particulate material in the mold frame by the consolidation load plate, so that the direction of motion of the particulate material during consolidation is toward the plug, where

$$L_c = F\gamma_1 A_1 B$$

and

F = overpressure factor,
$\gamma_1$ = bulk density of the particulate material being tested, and
$A_1$ = average cross sectional area of the test cell ;

and wherein, before the step of inverting the test cell, the method is further characterized by the steps of continuing to apply the consolidation load for an interval of time equal to how long the particulate material in the full-size container has been at rest, and removing the mold frame and the particulate material contained in it from the test cell ; and, after the step of noting the downward load, the method is further characterized by the step of determining whether the noted load at which failure occurred is less than

$$L_F = (\frac{B}{D} - 1) CA_1\gamma_1$$

whereby, if it is, then flow from the full-size container will occur by gravity, but if it is not, then flow from the full-size container will not occur by gravity.

16. The method of Claim 10 wherein the flow-related property is the unconfined yield strength of the particulate material and wherein, in the consolidating step the downward pressure $\sigma_c$ is given by

$$\sigma_c = \frac{L_c}{A_1} + \gamma_1 C$$

where

$L_c$ = consolidation load
$A_1$ = average cross sectional area of the test cell and
$\gamma_1$ = bulk density of the particulate material ;

and, after the step of noting the load, the method is further characterized by the step of calculating the unconfined yield strength by the equation

$$f_c = \frac{D}{h} (\gamma_1 + \frac{L_F}{A_1 C})$$

where h = 1.1.

17. Apparatus for use in ascertaining the flow properties of a particulate material, comprising : a hollow test cell that includes a frustrated wedge section defined by an inwardly-facing surface conforming to a section of a wedge, said frustrated wedge section having a smaller end and a larger end ; and, a plug located adjacent to and spanning the larger end of the frustrated wedge section ; but slightly narrower than it, so as to be freely movable a limited distance into the space within the frustrated wedge section ; said plug having a concave trough-like inwardly-facing surface.

18. The apparatus of Claim 17 wherein the vertex semi-angle of the frustrated wedge section is in the range between a minimum of 4 degrees and a maximum of $\theta_p$, where $\theta_p$ is the largest angle compatible with the mass-flow stress field in the test cell.

## Revendications

1. Un procédé d'essai au banc pour déterminer une propriété d'écoulement d'un matériau particulaire,

12

comprenant les étapes suivantes :

a) on établit une cellule d'essai creuse qui comprend une partie conique définie par une surface tournée vers l'intérieur conforme à une partie de hauteur C d'un cône, la partie conique présentant une extrémité de plus petit diamètre et une extrémité de plus grand diamètre D, la cellule comprenant en outre un bouchon obturant l'extrémité de plus grand diamètre de la partie conique, tout en étant de diamètre légèrement inférieur à ce dernier, de manière à pouvoir être déplacé librement axialement sur une distance limitée dans l'espace qui se situe à l'intérieur de la partie conique, le bouchon présentant une surface tournée vers l'intérieur concave ;

b) on place la cellule d'essai sur une surface de support de manière que l'extrémité de plus grand diamètre de la partie conique se trouve en dessous de son extrémité de plus petit diamètre, le bouchon étant disposé dans la position décrite ci-dessus, adjacente à l'extrémité de plus grand diamètre de la partie conique ;

c) on remplit la cellule d'essai complètement avec le matériau particulaire ;

d) on tasse le matériau particulaire dans la cellule d'essai en appliquant une pression dirigée vers le bas au niveau du plus petit diamètre de la partie conique, de manière que la direction du mouvement du matériau particulaire pendant le tassement soit orientée vers le bouchon ;

e) on inverse la cellule d'essai avec le bouchon et le matériau particulaire tassé maintenus dans la cellule, de manière qu'après inversion l'extrémité de plus grand diamètre de la section conique se trouve au-dessus de l'extrémité de plus petit diamètre ;

f) on applique une charge orientée vers le bas et croissant progressivement vers le bouchon de manière à produire un mouvement de dislocation du matériau particulaire vers l'extrémité de plus petit diamètre de la partie conique ; et

g) on note la charge à laquelle la dislocation apparaît.

2. Le procédé de la revendication 1, dans lequel la propriété d'écoulement est le diamètre minimum $B_{MIN}$ d'un orifice formé dans le fond d'un conteneur grandeur nature dans lequel doit se produire un écoulement du matériau particulaire sous l'effet de la gravité seulement et dans lequel l'étape de tassement du matériau particulaire est caractérisée en outre par l'étape suivant laquelle on applique une pression au matériau particulaire au niveau de l'extrémité de plus petit diamètre de la partie conique, pression égale à la pression de tassement à la position correspondante dans le conteneur grandeur nature et dans lequel, à la suite de l'étape dans laquelle on note la charge orientée vers le bas, la méthode est caractérisée en outre par l'étape de calcul du diamètre minimum auquel l'écoulement intervient, à l'aide de l'équation :

$$B_{MIN} = \frac{L_F D}{C A_1 \gamma_1} + D$$

où

$L_F$     est la charge orientée vers le bas, notée,

$A_1$     est la surface moyenne de la section transversale de la cellule d'essai et,

$\gamma_1$     est la densité du matériau particulaire en vrac.

3. Le procédé de la revendication 2 dans lequel le matériau particulaire a été laissé au repos dans le conteneur grandeur nature pendant un temps substantiel, et dans lequel l'étape de tassement est caractérisée en outre par l'application de la pression de tassement pendant un intervalle de temps égal à celui pendant lequel le matériau particulaire a été laissé au repos dans le conteneur grandeur nature.

4. Le procédé de la revendication 1 dans lequel la propriété d'écoulement concerne la capacité du matériau particulaire à s'écouler ou non, après avoir été laissé au repos pendant quelque temps, d'un conteneur grandeur nature qui exige un écoulement par gravité pour la décharge et qui comprend un orifice de sortie de diamètre B dans son fond ; et dans lequel l'étape de tassement du matériau particulaire dans la cellule d'essai par application d'une pression orientée vers le bas est en outre caractérisée par l'étape suivant laquelle on applique une charge de tassement $L_c$ distribuée uniformément sur le matériau particulaire à l'extrémité de plus petit diamètre de la partie conique, de façon que la direction de mouvement du matériau particulaire pendant la consolidation soit orientée vers le bouchon, où

$$L_c = \frac{1}{2} F \gamma_1 A_1 B$$

où

F =     facteur de surpression,

$\gamma_1$ =     densité du matériau particulaire en vrac à tester,

$A_1$ =     surface de section transversale moyenne de la cellule d'essai ;

et dans lequel le procédé de la revendication 1 est en outre caractérisé par l'étape suivant laquelle, à la suite de l'étape où l'on note la charge orientée vers le bas, on détermine si la charge notée à laquelle apparaît la dislocation est inférieure à

$$L_F = (\frac{B}{D} - 1)\, CA_1\gamma_1$$

ce par quoi, si c'est le cas, un écoulement par gravité du conteneur grandeur nature interviendra, alors que si ce n'est pas le cas, un écoulement par gravité du conteneur grandeur nature n'interviendra pas.

5. Le procédé de la revendication 4, dans lequel la charge de tassement $L_c$ est appliquée au matériau particulaire pendant un intervalle de temps égal à celui pendant lequel on a laissé le matériau particulaire au repos dans le conteneur grandeur nature.

6. Le procédé de la revendication 1, dans lequel la propriété d'écoulement concerne la capacité du matériau particulaire à s'écouler ou non, après avoir été laissé au repos pendant quelque temps, d'un conteneur grandeur nature qui demande un écoulement par gravité pour se décharger et, qui présente un orifice de sortie de diamètre B dans son fond ; et avant l'étape de remplissage de la cellule d'essai, caractérisé en outre par l'étape qui consiste à disposer un anneau de moulage creux et présentant une surface interne cylindrique de diamètre égal au diamètre de l'extrémité de plus petit diamètre de la partie conique ; et avant l'étape de remplissage de la cellule d'essai, caractérisé en outre par l'étape suivant laquelle on dispose l'anneau de moulage au-dessus et coaxialement avec la cellule d'essai pour former une extension vers le haut de cette cellule ; et après l'étape de remplissage de la cellule d'essai, caractérisé en outre par les étapes de remplissage de l'anneau de moulage avec le matériau particulaire et de mise en place d'un disque de charge de tassement présentant un diamètre extérieur légèrement inférieur à celui du diamètre intérieur de l'anneau de moulage, par-dessus le matériau particulaire dans l'anneau de moulage ; et dans lequel l'étape de tassement du matériau particulaire est obtenue par application d'une charge de tassement $L_c$ distribuée uniformément sur la surface du matériau particulaire dans l'anneau de moulage par le disque de charge de consolidation, de manière que la direction de mouvement du matériau particulaire pendant le tassement soit orientée vers le bouchon, où

$$L_c = \frac{1}{2}\, F\gamma_1 A_1 B$$

et

F =     facteur de surpression,

$\gamma_1$ =     densité en vrac du matériau particulaire à tester,

$A_1$ =     surface moyenne de section transversale de la cellule d'essai ;

et dans lequel, avant l'étape de renversement de la cellule d'essai, le procédé est caractérisé en outre par les étapes consistant à continuer l'application de la charge de tassement pendant un intervalle de temps égal à celui pendant lequel le matériau particulaire a été maintenu au repos dans le conteneur grandeur nature, et on enlève l'anneau de moulage et le matériau particulaire contenu dans cet anneau de la cellule d'essai ; et après l'étape de relevé de la charge orientée vers le bas, le procédé est en outre caractérisé par l'étape de détermination si la charge relevée au moment où la dislocation intervient est inférieure à

$$L_F = (\frac{B}{D} - 1)\, CA_1\gamma_1$$

ce par quoi, si c'est le cas, un écoulement par gravité à partir du conteneur grandeur nature interviendra, mais si ce n'est pas le cas, un écoulement par gravité du conteneur grandeur nature n'interviendra pas.

7. Le procédé de la revendication 1, dans lequel la propriété d'écoulement est la résistance à l'écoulement non confiné du matériau particulaire et dans lequel, dans l'étape de tassement, la pression orientée vers le bas $\sigma_c$ est donné par :

$$\sigma_c = \frac{L_c}{A_1} + \frac{1}{2}\gamma_1 C$$

où

$L_c =$      charge de consolidation

$A_1 =$      surface de section transversale moyenne de la cellule d'essai

$\gamma_1 =$      densité du matériau particulaire en vrac ;

et, après l'étape de relevé de la charge, le procédé est caractérisé en outre par l'étape de calcul de la résistance à l'écoulement non confiné par l'équation :

$$f_c = \frac{D}{h}\left(\gamma_1 + \frac{L_F}{A_1 C}\right)$$

où h = 2,1.

8. Appareil pour la détermination des propriétés d'écoulement d'un matériau particulaire, comprenant : une cellule d'essai creuse qui comprend une partie conique définie par une surface tournée vers l'intérieur et conforme à une partie d'un cône, cette partie conique présentant une extrémité de plus petit diamètre et une extrémité de plus grand diamètre ; et un bouchon disposé en adjacence à la partie de plus grand diamètre de ladite partie conique et recouvrant celle-ci, ce bouchon étant cependant de diamètre légèrement plus petit que cette extrémité de manière à pouvoir se déplacer librement et axialement sur une distance limitée dans l'espace intérieur à cette partie conique, ce bouchon présentant une surface tournée vers l'intérieur concave.

9. L'appareil de la revendication 8, dans lequel le demi-angle au sommet de la partie conique est comprise entre un minimum de 4° et un maximum de $\theta_c$, où $\theta_c$ est l'angle le plus grand compatible avec le champ de contrainte écoulement-masse dans la cellule d'essai.

10. Un procédé d'essai au banc pour déterminer une propriété d'écoulement d'un matériau particulaire, comprenant les étapes suivantes :

a) on établit une cellule d'essai creuse qui comprend une partie en coin tronqué définie par une surface tournée vers l'intérieur se conformant à une partie de hauteur C d'un coin, la partie en coin tronqué présentant une petite extrémité et une grande extrémité de largeur D, et qui comprend en outre un bouchon disposé sur la grande extrémité de la partie en coin tronqué de manière à l'obturer tout en étant légèrement plus petite que celle-ci, de manière à être librement déplaçable sur une distance limitée dans l'espace qui se trouve à l'intérieur de la partie en coin tronqué, le bouchon présentant une surface orientée vers l'intérieur en forme d'auge concave ;

b) on place la cellule d'essai sur une surface de support avec une grande extrémité de la partie en coin tronqué disposée en dessous de sa petite extrémité et avec le bouchon disposé dans la position mentionnée ci-dessus adjacente à la grande extrémité de la partie en coin tronqué ;

c) on remplit la cellule d'essai complètement avec le matériau particulaire ;

d) on tasse le matériau particulaire dans la cellule d'essai en appliquant une pression orientée vers le bas et vers la petite extrémité de la partie en coin tronqué de manière que la direction de mouvement du matériau particulaire pendant le tassement soit orientée vers le bouchon,

e) on renverse la cellule d'essai avec le bouchon et le matériau particulaire tassé dans cette cellule, de manière qu'après renversement la grande extrémité de la partie en coin tronqué soit au-dessus de la petite extrémité,

f) on applique une charge orientée vers le bas et croissant progressivement sur le bouchon pour provoquer un mouvement de dislocation du matériau particulaire vers la petite extrémité de la partie en coin tronqué et,

g) on note la charge vers le bas à laquelle la dislocation apparaît.

11. Le procédé de la revendication 10, dans lequel la propriété d'écoulement est la largeur minimum $B_{MIN}$ d'un orifice de sortie du fond d'un conteneur grandeur nature duquel un écoulement de particule doit pouvoir s'opérer sous l'action de la seule gravité et dans lequel l'étape de tassement du matériau particulaire est caractérisée en outre par l'étape qui consiste à appliquer une pression au matériau particulaire, à la petite extrémité de la partie en coin, égale à la pression de tassement à la position correspondant dans le conteneur grandeur nature et dans lequel, après le relevé de la charge vers le bas, le procédé est caractérisé en outre par le calcul de la largeur minimum à laquelle un écoulement intervient à l'aide de l'équation :

EP 0 252 927 B1

$$B_{MIN} = \frac{L_F D}{CA_1\gamma_1} + D$$

où

$L_F$ est la charge vers le bas relevée,

$A_1$ est la surface de section transversale moyenne de la cellule d'essai et

$\gamma_1$ est la densité du matériau particulaire en vrac.

12. Le procédé de la revendication 11, dans lequel le matériau particulaire est laissé au repos dans le conteneur grandeur nature pendant un temps substantiel, et dans lequel l'étape de tassement est caractérisé en outre par l'application d'une pression de tassement pendant un intervalle de temps égal au temps pendant lequel le matériau particulaire est resté au repos dans le conteneur grandeur nature.

13. Le procédé de la revendication 10, dans lequel la propriété d'écoulement est constituée par la capacité du matériau particulaire à s'écouler ou non, après avoir été mis au repos pendant quelque temps, d'un conteneur grandeur nature qui exige un écoulement par gravité pour se décharger et qui présente un orifice de sortie de largeur B dans son fond, et dans lequel l'étape de tassement du matériau particulaire dans la cellule d'essai par application d'une pression orientée vers le bas est caractérisée en outre par l'application d'une charge de tassement $L_c$ distribuée uniformément sur le matériau particulaire à la petite extrémité de la partie en coin tronqué, de manière que la direction de mouvement du matériau particulaire pendant le tassement soit orientée vers le bouchon, où

$$L_c = F\gamma_1 A_1 B$$

où

$F =$ facteur de surpression

$\gamma_1 =$ densité du matériau particulaire en vrac à tester,

$A_1 =$ surface de section transversale moyenne de la cellule d'essai,

et dans lequel le procédé de la revendication 11 est caractérisé en outre, après le relevé de la charge vers le bas, par la détermination de ce que la charge relevée à la dislocation est inférieure ou non à :

$$L_F = (\frac{B}{D} - 1) CA_1\gamma_1$$

ce par quoi, si c'est le cas, un écoulement par gravité du conteneur grandeur nature interviendra, mais si ce n'est pas le cas, un écoulement par gravité du conteneur grandeur nature n'interviendra pas.

14. Le procédé de la revendication 13, dans lequel la charge de tassement $L_c$ est appliquée au matériau particulaire pendant un intervalle de temps égal à celui pendant lequel le matériau particulaire doit rester au repos dans le conteneur grandeur nature.

15. Le procédé de la revendication 10, dans lequel la propriété d'écoulement concerne la capacité du matériau particulaire à s'écouler, après avoir été mis au repos pendant quelque temps, d'un conteneur grandeur nature qui demande un écoulement par gravité pour se décharger et qui présente un orifice de largeur B dans son fond, caractérisé en outre, avant le remplissage de la cellule d'essai, par l'utilisation d'un cadre de moulage creux et présentant une surface interne rectangulaire de dimension égale à celle de la petite extrémité de la partie en coin tronqué et, avant le remplissage de la cellule d'essai, caractérisé en outre par l'installation du cadre de moulage par-dessus et en repérage avec la cellule d'essai de manière à former une extension vers le haut de cette cellule, et, après le remplissage de la cellule d'essai, caractérisé en outre par le remplissage du cadre de moulage avec le matériau particulaire et l'installation d'une plaque de charge de tassement présentant des dimensions extérieures légèrement inférieures aux dimensions intérieures du cadre de moulage par-dessus le matériau particulaire dans le cadre de moulage, et dans lequel le tassement du matériau particulaire est obtenu par application d'une charge de tassement $L_c$ distribuée uniformément au-dessus du matériau particulaire dans le cadre de moulage par la plaque de charge de tassement, de manière que la direction de déplacement du matériau particulaire pendant le tassement soit dirigée vers le bouchon, ou

$$L_c = F\gamma_1 A_1 B$$

16

et

F = facteur de supression,
$\gamma_1$ = densité du matériau particulaire à tester en vrac, et
$A_1$ = surface de section transversale moyenne de la cellule d'essai,

et dans lequel, avant le renversement de la cellule d'essai, le procédé est caractérisé en outre par un maintien de l'application de la charge de tassement pendant un intervalle de temps égal à celui pendant lequel le matériau particulaire est resté au repos dans le conteneur grandeur nature, et l'enlèvement du cadre de moulage du matériau particulaire contenu dans ce cadre de la cellule d'essai et, après le relevé de la charge orientée vers le bas, le procédé est caractérisé en outre par l'étape dans laquelle on détermine si la charge relevée à la dislocation est inférieure à

$$L_F = (\frac{B}{D} - 1)\, CA_1\gamma_1$$

ce par quoi, si c'est le cas, un écoulement par gravité du conteneur grandeur nature interviendra et si ce n'est pas le cas, un écoulement par gravité du conteneur grandeur nature n'interviendra pas.

16. Le procédé de la revendication 10, dans lequel la propriété d'écoulement est la résistance à l'écoulement non confiné du matériau particulaire et dans lequel, dans l'étape de tassement, la pression vers le bas $\sigma_c$ est donnée par :

$$\sigma_c = \frac{L_c}{A_1} + \gamma_1 C$$

ou

$L_c$ = charge de tassement
$A_1$ = surface de la section transversale moyenne de la cellule d'essai et
$\gamma_1$ = densité du matériau particulaire ;

et, après le relevé de la charge, le procédé est caractérisé par le calcul de la résistance à l'écoulement non confiné du matériau à l'aide de l'équation

$$f_c = \frac{D}{h}\, (\gamma_1 + \frac{L_F}{A_1 C})$$

ou h = 1,1.

17. Appareil pour la détermination des propriétés d'écoulement d'un matériau particulaire comprenant : une cellule d'essai creuse qui comprend une section en coin tronqué définie par une surface tournée vers l'intérieur en forme d'une partie de coin, ladite partie tronquée présentant une petite extrémité et une grande extrémité, et un bouchon adjacent et recouvrant la grande extrémité de la partie en coin tronqué mais légèrement plus étroit qu'elle de manière à pouvoir se déplacer librement sur une distance limitée dans l'espace intérieur à la partie en coin tronqué ; ledit bouchon présentant une surface tournée vers l'intérieur en forme d'angle concave.

18. L'appareil de la revendication 17, dans lequel le demi-angle au sommet de la partie en coin tronqué est compris entre un minimum de 4 degrés et un maximum de $\theta_p$, dans lequel $\theta_p$ est le plus grand angle compatible avec le champ de contraintes écoulement-masse dans la cellule d'essai.

**Patentansprüche**

1. Verfahren zum werkbankmaßstäblichen Testen zur Bestimmung einer mit der Strömung in Beziehung stehenden Eigenschaft eines teilchenförmigen Materials, wobei folgende Schritt vorgesehen sind :
a) Vorsehen einer hohlen Testzelle, die einen konischen Abschnitt aufweist, und zwar definiert durch eine nach innen weisende Oberfläche, die einem Konusabschnitt mit der Höhe C entspricht, wobei der Konusabschnitt ein Ende mit einem kleineren Durchmesser und ein Ende mit einem größeren Durchmesser D aufweist, und wobei ferner ein Stopfen benachbart zu und das Ende mit dem größeren Durchmesser des konischen Abschnitts überspannend angeordnet ist, aber mit einem etwas kleineren Durchmesser als dieses endet, um so axial frei beweglich über einen begrenzten Abstand in den Raum innerhalb des konischen Abschnitts zu sein, wobei der Stopfen eine konkave, nach innen weisende Oberfläche besitzt;

b) Anordnen der Testzelle auf einer Tragoberfläche, wobei das einen größeren Durchmesser besitzende Ende des konischen Abschnitts unterhalb seines einen kleineren Durchmesser besitzenden Endes liegt und wobei der Stopfen in seiner zuvor erwähnten Lage benachbart zu den einen größeren Durchmesser besitzenden Ende des konischen Abschnitts getragen ist;

c) Vollständiges Anfüllen der Testzelle mit dem teilchen förmigen Material;

d) Konsolidierung des teilchenförmigen Materials innerhalb der Testzelle durch Aufbringen eines nach unten gerichteten Drucks auf das einen kleineren Durchmesser besitzende Ende des konischen Abschnitts derart, daß die Bewegungsrichtung des teilchenförmigen Materials während der Konsolidierung zum Stopfen hin erfolgt;

e) Umkehren der Testzelle mit dem Stopfen und dem noch immer darin befindlichen konsolidierten, teilchenförmigen Material, derart, daß nach der Umkehr das einen größeren Durchmesser besitzende Ende des konischen Abschnitts oberhalb des einen kleineren Durchmesser besitzenden Endes angeordnet ist;

f) Anlegen einer allmählich ansteigenden, nach unten gerichteten Belastung an den Stopfen zur Erzeugung einer Ausfallbewegung des teilchenförmigen Materials zum einen kleineren Durchmesser besitzenden Ende des konischen Abschnitts hin; und

g) Feststellen der nach unten gerichteten Last, bei der der Ausfall auftritt.

2. Verfahren nach Anspruch 1, wobei die mit der Strömung in Beziehung stehende Eigenschaft der minimale Durchmesser $B_{MIN}$ eines Auslasses im Boden eines die volle Größe besitzenden Behälters ist, bei dem dip Strömung des teilchenförmigen Materials allein durch die Schwerkraft erfolgt; und wobei der Schritt des Konsolidierens des teilchenförmigen Materials ferner gekennzeichnet ist durch den Schritt des Anlegens eines Drucks an das teilchenförmige Material an dem einen kleineren Druchmesser aufweisenden Ende des konischen Abschnitts gleich dem Konsolidierungsdruck an der entsprechenden Stelle im die volle Größe besitzenden Behälter; und wobei darauffolgend auf den Schritt des Feststellens der nach unten gerichteten Last das Verfahren ferner gekennzeichnet ist durch den Schritt der Berechnung des minimalen Durchmessers bei dem Strömung auftritt gemäß der Gleichung

$$B_{MIN} = \frac{L_F D}{C A_1 \gamma_1} + D$$

dabei ist

$L_F$    die festgestellte nach unten gerichete Last,
$A_1$    die durchschnittliche Querschnittsfläche der Testzelle, und
$\gamma_1$    die Massendichte des teilchenförmigen Materials.

3. Verfahren nach Anspruch 2, wobei das teilchenförmige Material in dem die volle Größe besitzenden Behälter für eine merkliche Zeitquote, und wobei der Schritt des Konsolidierens ferner gekennzeichnet ist durch das Anlegen des Konsolidierungsdrucks für ein Zeitintervall gleich der Zeitdauer mit der das teilchenförmige Material in dem die volle Größe besitzenden Behälter ruhte.

4. Verfahren nach Anspruch 1, wobei die mit der Strömung in Beziehung stehende Eigenschaft diejenige ist, ob das teilchenförmige Material fließen wird oder nicht, nachdem es einige Zeit in Ruhe war, und zwar aus einem die volle Größe besitzenden Behälter, der für die Abgabe Schwerkraftfluß erforderlich macht, und der einen Auslaßdurchmesser B in seinem Boden besitzt, und wobei der Schritt des Konsolidierens des teilchenförmigen Materials innerhalb der Testzelle durch Anlegen eines nach unten gerichteten Drucks ferner gekennzeichnet ist durch den Schritt des Anlegens einer konsolidierten Last $L_c$ gleichförmig verteilt über das teilchenförmige Material an dem einen kleineren Durchmesser aufweisenden Ende des konischen Abschnitts derart, daß dip Bewegungsrichtung des teilchenförmigen Materials während der Konsolidierung zum Stopfen hin erfolgt, wobei

$$L_c = \frac{1}{2} F \gamma_1 A_1 B$$

dabei ist:

F = Überdruckfaktor,

$\gamma_1$ = Massendichte des getesteten teilchenförmigen Materials,

$A_1$ = durchschnittliche Querschnittsfläche der Testzelle ;

und wobei das Verfahren nach Anspruch 1 ferner gekennzeichnet ist durch den Schritt, daß darauffolgend auf den Schritt des Feststellens der nach unten gerichteten Kraft der Schritt der Bestimmung folgt, ob die festgestellte Last, bei der der Ausfall auftrat kleiner ist als

$$L_F = (\frac{B}{D} - 1)\, CA_1\gamma_1$$

wodurch, wenn dies der Fall ist, der Fluß von dem die volle Größe besitzenden Behälter durch Schwerkraft auftritt, aber, wenn dies nicht der Fall ist, der Fluß von dem die volle Größe besitzenden Behälter nicht durch Schwerkraft auftritt.

5. Verfahren nach Anspruch 4, wobei die Konsolidierungslast $L_c$ an das teilchenförmige Material für ein Zeitintervall angelegt wird, welches gleich dem Zeitraum ist, für den das teilchenförmige Material in dem die volle Größe besitzenden Behälter im Ruhezustand war.

6. Verfahren nach Anspruch 1, wobei die mit der Strömung in Beziehung stehende Eigenschaft darin besteht, ob das teilchenförmige Material fließen wird, oder ob das teilchenförmige Material nicht fließen wird, und zwar nachdem es für eine Zeitspanne in Ruhe war, und zwar aus einem die volle Größe besitzenden Behälter, der zur Abgabe den Schwerkraftfluß benötigt und der einen Auslaß mit dem Durchmesser B in seinem Boden besitzt, wobei ferner vor dem Schritt des Füllens der Testzelle in kennzeichnender Weise der Schritt des Vorsehens eines Formrings vorgesehen ist, der hohl ist und der eine zylindrische Innenoberfläche besitzt mit einem Durchmesser gleich dem Durchmesser des einen kleineren Durchmesser besitzenden Endes des konischen Abschnittes, und wobei ferner vor dem Schritt des Füllens der Testzelle kennzeichnenderweise der Schritt des Ruhens des Formringes auf der Oberseite und koaxial mit der Testzelle vorgesehen ist, um eine nach oben gerichtete Verlängerung derselben zu bilden, wobei schließlich nach dem Schritt des Füllens der Testzelle ferner kennzeichnenderweise die folgenden Schritte vorgesehen sind : Füllen des Formringes mit dem teilchenförmigen Material und Anordnen einer Konsolidierungslastscheibe mit einem Außendurchmesser etwas kleiner als dem Innendurchmesser des Formringes auf der Oberseite des teilchenförmigen Materials in dem Formring ; wobei schließlich der Schritt des Konsolidierens des teilchenförmigen Materials erreicht wird durch Aufbringen einer Konsolidierungslast $L_c$ gleichförmig verteilt über die Oberseite des teilchenförmigen Materials im Formring durch die Konsolidierungslastscheibe derart, daß die Richtung der Bewegung des teilchenförmigen Materials während der Konsolidierung zum Stopfen hin erfolgt, wobei

$$L_c = \frac{1}{2}\, F\gamma_1 A_1 B$$

dabei ist :

F = Überdruckfaktor,

$\gamma_1$ = Massendichte des im Test befindlichen teilchenförmigen Materials

$A_1$ = durchschnittliche Querschnittfläche der Testzelle ;

und wobei ferner vor dem Schritt des Umkehrens der Testzelle das Verfahren gekennzeichnet ist durch die folgenden Schritte : Fortsetzen des Aufbringens der Konsolidierungslast für ein Zeitintervall gleich der Zeitspanne über die hinweg das teilchenförmige Material in dem die volle Größe besitzenden Behälter ruhte und Entfernung des Formringes und des teilchenförmigen Materials enthalten darin aus der Testzelle, wobei schließlich nach dem Schritt der Feststellung ob die aufgezeichnete Last, bei der der Ausfall auftrat kleiner ist als

$$L_f = (\frac{B}{D} - 1)\, CA_1\gamma_1$$

wodurch dann, wenn dies der Fall ist, der Fluß aus dem die volle Größe besitzenden Behälter durch Schwerkraft auftreten wird, während dann, wenn dies nicht der Fall ist, der Fluß von dem die volle Größe besitzenden Behälter nicht durch Schwerkraft auftreten wird.

7. Verfahren nach Anspruch 1, wobei die mit der Strömung in Beziehung stehende Eigenschaft die nicht beschränkte Streckfähigkeit des teilchenförmigen Materials ist und wobei im Konsolidierungsschritt der nach unten gerichtete Druck sigma$_c$ gegeben ist durch

$$\sigma_c = \frac{L_c}{A_1} + \frac{1}{2}\gamma_1 C$$

dabei ist :

$L_c$ = Konsolidierungslast,

$A_1$ = durchschnittliche Querschnittsfläche der Testzelle,

$\gamma_1$ = Massendichte des teilchenförmigen Materials ;

und wobei ferner nach dem Schritt der Aufzeichnung der Last das Verfahren ferner gekennzeichnet ist durch den Schritt des Berechnens der nicht eingeschränkten Streckfestigkeit durch die Gleichung,

$$f_c = \frac{D}{h}(\gamma_1 + \frac{L_F}{A_1 C})$$

wobei h = 2.1.

8. Vorrichtung zur Verwendung bei der Bestimmung der Strömungseigenschaften eines teilchenförmigen Materials, wobei folgendes vorgesehen ist : eine hohle Testzelle, die einen konischen Abschnitt aufweist, definiert durch eine nach innen weisende Oberfläche die einem Abschnitt eines Konus entspricht, wobei der konische Abschnitt ein einen kleineren Durchmesser besitzendes Ende und ein einen größeren Durchmesser besitzendes Ende aufweist ; und einen Stopfen angeordnet benachbart zu und das den größeren Durchmesser besitzende Ende des konischen Abschnittes überspannenden, der aber einen etwas kleineren Durchmesser als das Ende aufweist, um so frei axial bewegbar mit einem begrenzten Abstand in den Raum innerhalb des konischen Abschnitts zu sein, wobei der Stopfen eine konkave, nach innen weisende Oberfläche besitzt.

9. Vorrichtung nach Anspruch 8, wobei der Scheitelhalbwinkel des konischen Abschnitts im Bereich zwischen einem Minimum von 4° und einem Maximum von theta$_c$ liegt, wobei theta$_c$ der größte Winkel ist, der mit dem Massenströmungsbeanspruchfeld in der Testzelle kompatibel ist.

10. Verfahren zum Testen auf Werkbankmaßstab zur Feststellung einer mit der Strömung in Beziehung stehenden Eigenschaft eines teilchenförmigen Materials, wobei die folgenden Schritte vorgesehen sind :

a) Vorsehen einer hohlen Testzelle, die einen Keilstumpfabschnitt aufweist, und zwar definiert durch eine nach innen weisende Oberfläche entsprechend einem Abschnitt der Höhe C eines Keils, wobei der Keilstumpfabschnitt ein kleines Ende und ein größeres Ende der Breite D aufweist, wobei ferner ein Stopfen vorgesehen ist, der benachbart zu und das größere Ende des Keilstumpfabschnitts überspannend vorgesehen ist, der aber ferner etwas schmäler ist, als dieses, um so über einen begrenzten Abstand frei in den Raum innerhalb des Keilstumpfabschnitts bewegbar zu sein, wobei der Stopfen eine konkave, druckartige nach innen weisende Oberfläche besitzt ;

b) Anordnen der Testzelle auf einer Tragoberfläche mit dem größeren Endes des Keilstumpfabschnittes unterhalb seines schmaleren Endes und mit dem Stopfen getragen an seiner oben erwähnten Stelle benachbart zum größeren Ende des Keilstumpfabschnitts ;

c) Vollständiges Anfüllen der Testzelle mit dem teilchenförmigen Material ;

d) Konsolidierung des teilchenförmigen Materials innerhalb der Testzelle durch Aufbringen eines nach unten gerichteten Druckes auf das kleinere Ende des Keilstumpfabschnittes derart, daß die Bewegungsrichtung des teilchenförmigen Materials während der Konsolidierung zum Stopfen hin erfolgt ;

e) Umkehren der Testzelle mit dem Stopfen und dem noch immer darin befindlichen konsolidierten teilchenförmigen Material derart, daß nach der Umkehr das größere Ende des Keilstumpfabschnitts oberhalb seines schmaleren Endes sich befindet ;

f) Aufbringen einer sich graduell erhöhenden nach unten gerichteten Belastung auf den Stopfen zur Erzeugung der Ausfallbewegung des teilchenförmigen Materials zum kleineren Ende des Keilstumpfabschnitts hin ; und

g) Aufzeichnung der nach unten gerichteten Last bei der der Ausfall auftritt.

11. Verfahren nach Anspruch 10 wobei die mit der Strömung in Beziehung stehende Eigenschaft die minimale Breite $B_{min}$ eines Auslasses im Boden eines die volle Größe besitzenden Behälters ist, bei der der Fluß oder die Strömung des teilchenförmigen Materials allein durch die Schwerkraft

$$B_{MIN} = \frac{L_F D}{CA_1\gamma_1} + D$$

wobei

$L_f$     die aufgezeichnete, nach unten gerichtete Last,

$A_1$     die durchschnittliche Querschnittsfläche der Testzelle,

$\gamma_1$     die Massendichte des teilchenförmigen Materials ist.

12. Verfahren nach Anspruch 11, wobei das teilchenförmige Material in dem die volle Größe besitzenden Behälters für eine merkliche Zeit in Ruhe sich befand und wobei der Schritt der Konsolidierung ferner gekennzeichnet ist durch Anlegen des Konsolidierungsdruckes für ein Zeitintervall gleich der Zeit für die das teilchenförmige Material des die volle Größe besitzenden Behälters im Ruhezustand sich befand.

13. Verfahren nach Anspruch 10, wobei die mit der Strömung in Beziehung stehende Eigenschaft die ist, ob das teilchenförmige Material fließen wird oder ob es nicht fließen wird, und zwar nachdem es für eine gewisse Zeit sich in Ruhe befand und zwar aus einem die volle Größe besitzenden Behälter heraus der Schwerkraftfluß für die Abgabe benötigt, und der einen Auslaß mit der Breite B in seinem Boden besitzt ; und wobei ferner der Schritt des Konsolidierens des teilchenförmigen Materials innerhalb der Testzelle durch Anlegen eines nach unten gerichteten Drucks ferner gekennzeichnet ist durch den Schritt des Anlegens einer Konsolidierungslast $L_c$ gleichförmig verteilt über das teilchenförmige Material an dem kleineren Ende des Keilstumpfabschnitts derart, daß die Bewegungsrichtung des teilchenförmigen Materials während der Konsolidierung zum Stopfen hin erfolgt, wobei folgedes gilt :

$$L_c = F\gamma_1 A_1 B$$

dabei ist

$F =$     Überdruckfaktor

$\gamma_1$     Massendichte des teilchenförmigen Materials im Test

$A_1$     durchschnittliche Querschnittsfläche der Testzelle ;

und wobei das Verfahren des Anspruchs 11 ferner gekennzeichnet ist durch den auf den Schritt des Aufzeichnens der nach unten gerichteten Last folgenden Schritts der Bestimmung ob die aufgezeichnete Last bei der der Ausfall auftritt kleiner ist als

$$L_F = (\frac{B}{D} - 1)\, CA_1\gamma_1$$

wodurch dann, wenn dies der Fall ist, die Strömung aus dem die volle Größe besitzenden Behälter durch Schwerkraft erfolgt, während, wenn dies nicht der Fall ist, die Strömung aus dem die volle Größe besitzenden Behälter nicht durch Schwerkraft erfolgt.

14. Verfahren nach Anspruch 13, wobei die Konsolidierungslast $L_c$ an das teilchenförmige Material für ein Zeitintervall angelegt wird, welches gleich der Zeitspanne ist, für die das teilchenförmige Material in dem die volle Größe besitzenden Behälter sich im Ruhezustand befand.

15. Verfahren nach Anspruch 10, wobei die mit der Strömung in Beziehung stehende Eigenschaft darin besteht, ob das teilchenförmige Material fließen wird oder nicht fließen wird, und zwar nachdem es sich für eine gewisse Zeit in Ruhe befand und zwar aus dem die volle Größe besitzenden Behälter der Schwerkraftfluß für den Abfluß erforderlich macht und der einen Auslaß mit der Breite B in seinem Boden besitzt, und wobei ferner vor dem Schritt des Füllens der Testzelle kennzeichnenderweise der Schritt des Vorsehens eines Formrahmens vorgesehen ist, der hohl ist und der eine rechteckige Innenoberfläche aufweist und zwar von Dimensionen gleich denjenigen des kleineren Endes des Keilstumpfabschnitts ; und wobei weiterhin vor dem Schritt des Anfüllens der Testzelle kennzeichnenderweise der Schritt des Ruhens des Formrahmens auf der Oberseite der Testzelle und ausgerichtet mit dieser vorgesehen ist zur Bildung einer nach oben gerichteten Verlängerung derselben ; und wobei schließlich nach dem Schritt des Füllens der Testzelle kennzeichnenderweise die Schritte des Füllens des Formrings mit dem teilchenförmigen Material und des Anordnens einer Konsolidationslastplatte mit Außendimensionen etwas kleiner als die Innendimension des Formrahmens auf der Oberseite des teilchenförmigen Materials in dem Formrahmen vorgesehen ist ; und wobei schließlich der Schritt der Konsolidierung des teilchenförmigen Materials erreicht wird durch Anlegen einer Konsolidierungslast $L_c$ gleichförmig verteilt über die Oberseite des teilchenförmigen Materials im Formrahmen durch die Konsolidierungslastplatte derart, daß die Richtung der Bewegung des teilchenförmigen Materials während der Konsolidierung zum Stopfen hin erfolgt, wobei folgendes gilt :

$$L_c = F\gamma_1 A_1 B$$

dabei ist

F = Überdruckfaktor,

$\gamma_1$    Massendichte des teilchenförmigen Materials im Test

$A_1$    durchschnittliche Querschnittsfläche der Testzelle ;

und wobei ferner daß Verfahren vor dem Schritt des Umkehrens der Testzelle gekennzeichnet ist durch die Schritte der Fortsetzung des Anlegens der Konsolidierungslast für ein Zeitintervall gleich der Zeitspanne für die das teilchenförmige Material in dem die volle Größe besitzenden Behälter in Ruhe war und Entfernen des Formrahmens und des darin enthaltenen teilchenförmigen Materials aus der Testzelle ; und wobei nach dem Schritt des Aufzeichnens der nach unten gerichteten Last das Verfahren ferner gekennzeichnet ist durch den Schritt der Bestimmung ob die aufgezeichnete Last bei der der Ausfall auftrat, kleiner ist als

$$L_F = (\frac{B}{D} - 1) CA_1\gamma_1$$

wodurch dann, wenn dies der Fall ist, der Fluß aus dem die volle Größe besitzenden Behälter durch Schwerkraft erfolgt, aber, wenn dies nicht der Fall ist, der Fluß aus dem die volle Größe besitzenden Behälters nicht durch Schwerkraft erfolgt.

16. Verfahren nach Anspruch 10, wobei die mit der Strömung in Beziehung stehende Eigenschaft die nicht eingeschränkte Streckfestigkeit des teilchenförmigen Materials ist und wobei in dem Konsolidierungsschritt der nach unten gerichtete Druck $\sigma_c$ gegeben ist durch

$$\sigma_c = \frac{L_c}{A_1} + \gamma_1 C$$

dabei ist

$L_c$    die Konsolidierungslast

$A_1$    die durchschnittliche Querschnittsfläche der Testzelle

$\gamma_1$    Massendichte des teilchenförmigen Materials

und wobei ferner das Verfahren nach dem Schritt der Aufzeichnung der Last gekennzeichnet ist durch den Schritt der Berechnung der nicht eingeschränken Streckfestigkeit durch die Gleichung

$$f_c = \frac{D}{h} (\gamma_1 + \frac{L_F}{A_1 C})$$

wobei h gleich 1,1.

17. Vorrichtung zur Verwendung bei der Feststellung der Strömungseigenschaften von teilchenförmigen Material, wobei folgendes vorgesehen ist : eine hohle Testzelle die einen Keilstumpfabschnitt aufweist und zwar definiert durch eine nach innen weisende Oberfläche entsprechend einem Keilabschnitt wobei der Keilstumpfabschnitt ein kleineres und ein größeres Ende besitzt ; und ein Stopfen angeordnet benachbart zu und das größere Ende des Keilstumpfabschnitts überspannend, wobei der Stopfen aber etwas schmäler ist als dieses, um sich frei über einen begrenzten Abstand in den Raum innerhalb des Keilstumpfabschnitts bewegen zu können, wobei der Stopfen eine konkave trogartige nach innen weisende Oberfläche besitzt.

18. Vorrichtung nach Anspruch 17, wobei der Scheitelhalbwinkel des Keilstupfabschnitts im Bereich zwischen einen Minimum zwischen 4 Grad und einem Maximum von $theta_p$ liegt, wobei der größte Winkel kompatibel mit dem Massenströmungsbeanspruchungsfeld in der Testzelle ist.

FIG. 1

FIG. 2

FIG. 3

FIG. 4

FIG. 5

FIG. 6